# EUROPÄISCHE PATENTSCHRIFT

(11) **EP 3 362 789 B1**
(45) Veröffentlichungstag und Bekanntmachung des Hinweises auf die Patenterteilung: **02.12.2020**
(21) Anmeldenummer: 16788641.5
(22) Anmeldetag: 12.10.2016
(51) Int. Cl.: G01N 33/02, G01N 35/02, G01N 21/359, G01N 35/04

(54) **VORRICHTUNG FÜR DIE AUTOMATISIERTE ANALYSE VON FESTSTOFFEN ODER FLUIDEN**
DEVICE FOR THE AUTOMATED ANALYSIS OF SOLIDS OR FLUIDS
DISPOSITIF D'ANALYSE AUTOMATIQUE DE MATIÈRES SOLIDES OU DE FLUIDES

(30) Priorität: 13.10.2015 DE 102015013140
(43) Veröffentlichungstag der Anmeldung: 22.08.2018
(73) Patentinhaber: Endress + Hauser Conducta GmbH+Co. KG, 70839 Gerlingen (DE)
(72) Erfinder: MANNHARDT, Joachim, 73569 Eschach (DE)
(74) Vertreter: Raunecker, Klaus Peter
(86) Internationale Anmeldenummer: PCT/EP2016/074502
(87) Internationale Veröffentlichungsnummer: WO 2017/064136

(56) Entgegenhaltungen:
- DE-B3- 10 332 800
- DE-T2- 68 921 284
- JP-A- H0 560 765
- US-B1- 7 053 373

## Beschreibung

Die Erfindung betrifft eine Vorrichtung für die automatisierte Analyse von Feststoffen oder Fluiden nach dem Oberbegriff des Anspruchs 1.

In nahezu allen verarbeitenden Industrien, wie beispielsweise der pharmazeutischen Industrie, der Nahrungsmittelindustrie oder der chemischen Industrie, wird die Qualitätskontrolle von Produkten häufig abseits des eigentlichen Produktionsprozesses in Laboren durchgeführt. Die Ursachen liegen hauptsächlich darin begründet, dass die Messsysteme zur chemischen Analyse sehr empfindlich sind und die Bedienung dieser eine grundlegende wissenschaftliche Fachkenntnis voraussetzt. Die Kosten komplex aufgebauter Messsysteme lassen einen Einsatz in Produktionsprozessen mit hohen Stückzahlen nicht zu. Oftmals befinden sich im Produktionsumfeld auch Gefahrenbereiche, in deren Umfeld ein Laborequipment nicht einsetzbar ist.

Bereits weit verbreitet sind spektroskopische Messmethoden zur quantitativen chemischen Analyse von Feststoffen und Fluiden. Optische Messeinrichtungen sind aus den Druckschriften WO 2014/080322 A1 und WO 2015/071706 A1 zur Messung der chemischen Zusammensetzung oder weiteren Probeneigenschaften von optischen dünnen Proben bekannt. Insbesondere Proben aus dem Agrarbereich sollen hiermit beispielsweise hinsichtlich ihres Proteinanteils bzw. Feuchtigkeitsgehaltes analysierbar sein.

Als technische Weiterentwicklung ist aus der DE 10 2013 006 948 A1 eine Vorrichtung für die automatisierte Analyse von Partikeln bekannt, insbesondere von solchen, die bei einer spanenden Materialbearbeitung von Holz anfallen. Beschrieben ist eine erste Station mit einer Dosiereinheit für ein Befüllen eines separaten Probentellers mit einer vorgegebenen Probenmenge, eine zweite Station mit einer Messvorrichtung für eine Analyse der Probe und eine dritte Station mit einer Entleer- und Reinigungsvorrichtung für einen Probenteller, sowie eine Transportvorrichtung für einen umlaufenden Transport des Probentellers in einer Richtung von einer Station zur nächsten, bis die erste Station erneut erreicht ist.

Aus der JP H05 60765 A, DE 689 21 284 T2 und DE 103 32 800 B3 sind ferner automatisierte Messsysteme bekannt, welche an den Messstationen Integrationskugeln nutzen, welche Strahlung von der Probenoberfläche einsammeln und dazu nahe der Oberfläche angeordnet sind.

Aufgabe der vorliegenden Erfindung ist es, eine Vorrichtung für die automatisierte Analyse von Feststoffen oder Fluiden hinsichtlich des Automatisierungsgrades weiterzubilden.

Diese Aufgabe wird gelöst durch die Vorrichtung mit den Merkmalen des Anspruchs 1. Die Unteransprüche betreffen vorteilhafte Ausführungsformen und Varianten der Erfindung.

Die Erfindung betrifft folglich eine Vorrichtung für die automatisierte Analyse von Partikeln oder Fluiden, mit der sehr rasch und quasi kontinuierlich getaktet Analysen vorgenommen werden können, insbesondere eine Bestimmung der chemischen Zusammensetzung der Proben.

Bei einem Umlauf wird die Probenkammer in der ersten Station mit der Probe verfüllt, in der zweiten Station erfolgt die eigentliche Analyse und in der dritten Station eine Entleerung und Reinigung. Die gereinigte Probenkammer steht erneut für ein Befüllen in der ersten Station zur Verfügung. Der Umlauf erfolgt taktweise, wobei der Takt durch die längste Verweildauer der Transportvorrichtung in einer der Stationen vorgegeben ist. Diese Zeitspanne ergibt sich in der Regel durch die Dauer der Analyse in der zweiten Station.

Für das Befüllen der zumindest einen Probenkammer ist eine Dosiereinheit vorgesehen, von der Proben in exakt gleicher Menge in eine Ausnehmung der Transportvorrichtung direkt eingebracht werden. Wesentlich beim Vorbereiten der Messprobe ist außer der Bereitstellung immer derselben Menge auch die Verteilung der Substanz in der Probenkammer selbst. Hierbei ist angedacht, dass einerseits eine gleichförmige Dicke eingestellt wird und andererseits eine gleichmäßige Verdichtung der Substanz stattfindet. Zur Analyse werden, insbesondere zur Messung fester Substanzen, auf diese Weise in der jeweiligen Probenkammer geeignet dünne Messproben präpariert. Für dünne, schichtartige Messproben eigenen sich besonders Probenkammern die aus flachen zylindrischen Ausnehmungen in der Transportvorrichtung selbst gebildet sind. Deckel- und bodenseitig bestehen die Probenkammern bevorzugt aus Material, welches transparent für die Messstrahlung ist.

Nach einem Vorschub einer gefüllten Probenkammer durch die Transportvorrichtung in die zweite Station erfolgt dort die Analyse. Dies kann eine chemische Analyse eines Feststoffes sein, gegebenenfalls jedoch auch eine Analyse gasförmiger bzw. flüchtiger Stoffe. Für eine Gasanalyse können weitere Messvorrichtungen mit gasführenden Anschlüssen zur Probenkammer angeordnet sein. Hierzu können an sich übliche Dichtungsmaßnahmen eingesetzt werden.

Die zweite Station mit zumindest einer Messvorrichtung für eine Analyse der in der Probenkammer befindlichen Probe ist ein Kugelmesssystem mit einer Integrationskugel in Form einer aus zwei Halbschalen bestehenden Photometerkugel.

Ein Kugelmesssystem kann als sphärischer Lichtkollektor aus Quarz oder Saphirglas gebildet und, bis auf gewisse Durchtrittsstellen für Lichtstrahlung, vollständig verspiegelt sein, um eine Lichtsammelwirkung zu erzielen. Der aus den Enden der Lichtwellenleiter austretende Lichtstrahl triff die verspiegelte Innenseite des Kugelmesssystems und wird von dort auf einen Sammelspiegel zurückreflektiert, welcher durch ein nicht verspiegeltes Fenster der Photometerkugel Licht auskoppelt. Dort kann beispielsweise eine Multimode-Faser zur Weiterleitung des Lichtes zu einem Detektor angeordnet sein. Ebenso ist angedacht, ein Auskoppeln des Lichtes ohne weitere Faserelemente direkt in einen Spektrometer vorzunehmen. Auf diese Weise ist die Photometerkugel eine Vorsatzeinheit zum Detektor. Mit einer integrierten kompakten Sensorik sind Prozessschnittstellen eingerichtet, die an bereits am Markt zur Verfügung stehende Datenmanagementlösungen angebunden werden können. Auf diese Weise lassen sich intelligente Prozessschnittstellen mit integrierten faseroptischen Spektralsensoren schaffen, die für den direkten Einsatz in Produktionslinien geeignet sind.

Das Kugelmesssystem eignet sich beispielsweise für Messungen mittels NIR-Spektroskopie, bei der die Probe über die Integrationskugel von vielen Seiten her beleuchtet wird. Hierbei sorgt die im Inneren verspiegelte Integrationskugel für eine homogene Lichtverteilung auf und in der Probe. Im Messsystem eignen sich zur Signalerfassung beispielsweise InGaAs-Detektoren mit einer hohen Sensitivität. Für quantitative Bestimmungen werden vorher, wie allgemein bei der Infrarotspektroskopie, Datensätze mit bekanntem Gehalt oder bekannten Konzentrationen des zu analysierenden Stoffes erstellt. Ebenso können auch Messsysteme für UV-Anwendungen in Betracht kommen.

Die Nahinfrarotspektroskopie basiert wie andere Schwingungsspektroskopien auf der Anregung von Molekülschwingungen durch elektromagnetische Strahlung im nahen Infrarotbereich. Bei der NIRS findet die Detektion bevorzugt im nahen Infrarot im Wellenlängenbereich von 760 - 2500 nm statt. NIR-Spektroskopie eignet sich beispielsweise zur Bestimmung des Wassergehaltes in vielerlei Produkten. Insbesondere können auch Qualitätsanalysen landwirtschaftlicher Produkte, wie beispielsweise Getreide, Mehl und Tierfutter zur Bestimmung von Feuchte, Protein- und Fettgehalt untersucht werden. Auch eine online Prozesskontrolle in der Nahrungsmittelindustrie oder bei chemischen und pharmazeutischen Produkten ist denkbar.

In der dritten Station erfolgt das Entleeren der Probenkammer, gegebenenfalls durch eine Absaugvorrichtung in einen oder mehrere Restbehälter. Auch die Restbehälter können für jede Probe austauschbar sein, so dass die Messreihen in ihrer zeitlichen Abfolge rekonstruierbar dokumentiert werden und einer der Messprobe zugrunde liegenden Charge zugeordnet werden können.

Der besondere Vorteil der Erfindung besteht darin, dass der Ablauf einer Analyse vollautomatisiert ist. Eines manuellen Eingriffs in den Ablauf der Analyse bedarf es üblicherweise nicht mehr. Insbesondere ermöglicht die erfindungsgemäße Vorrichtung eine in kurzen Zeitabständen ablaufende kontinuierlich getaktete Analyse. Der Dosiereinheit können die Probensubstanzen unmittelbar zugeführt werden. Damit ist die Qualitätskontrolle des Probenmaterials in einen Produktionsprozess einbindbar. Bei Abweichungen der Messwerte von den vordefinierten Sollwerten kann durch eine entsprechende Zuordnung der Probensubstanz zur jeweiligen Charge gegebenenfalls ohne zeitlichen Verzug in einen Produktionsprozess eingegriffen werden. Durch die Integration der Qualitätskontrolle in Produktionsabläufe wird eine Effizienzsteigerung sowie eine damit verbundene Reduzierung der Produktionskosten erzielt.

Ein weiterer Vorteil der Erfindung ergibt sich durch eine ausgesprochen kompakte Bauweise der Messvorrichtung. Insbesondere kann auch eine vorteilhafte Miniaturisierung des Kugelmesssystems realisiert werden, ohne die Einrichtungen zur Ein- und Auskopplung von Licht konstruktiv aufwändiger gestalten zu müssen. Derartige faseroptische Messkomponenten sowie geeignete Sensorsysteme und der damit zu erzielende Automatisierungsgrad tragen ganz wesentlich zur Steigerung eines wirtschaftlichen Betriebs bei.

In vorteilhafter Ausgestaltung der Erfindung kann die Transportvorrichtung einen Drehteller umfassen, in dem eine Probenkammer integriert ist oder mehrere Probenkammern integriert sind. Der Drehteller weist bevorzugt so viele Probenkammern auf, wie Taktzyklen in den Stationen vorhanden sind. Dabei wird zunächst in eine erste Probenkammer mittels einer Probenzuführung eine erste Messprobe, beispielsweise Milchpulver, Getreide oder sonst schütt- oder fließfähige Güter, eingebracht. Nachfolgend wird der Drehteller um ein gewisses Winkelmaß weitergedreht und der nächste Takt eingeleitet. Hierbei tritt die gefüllte Probenkammer in die Integrationskugel ein, welche bevorzugt auf der Ebene des Drehtellers von der Seite her gehaltert ist. In der Integrationskugel erfolgt dann beispielsweise eine Transmissionsmessung. Hierbei erfasst die Analyse auch das Innere eines zu vermessenden Gutes, also beispielsweise das Innere von Getreidekörnern zur Bestimmung des Eiweißgehalts. Im darauffolgenden Takt wird die Probe aus der Probenkammer, beispielsweise durch ein Absaugen, entfernt und die Probenkammer gereinigt, so dass diese wieder für die Aufnahme einer weiteren Messprobe zur Verfügung steht.

Wesentlich beim Messablauf ist dabei, dass bevorzugt eine getaktete Arbeitsweise der Analysevorrichtung gewählt wird. Hierdurch werden die unterschiedlichen Arbeitsschritte während des Stillstandes des Drehtellers vorgenommen. Hierdurch ist eine effiziente Abdichtung der einzelnen Probenräume zum Befüllen, Messen und Reinigen möglich.

Der Drehteller ist bevorzugt mit einer im Umfang gleichen Winkelteilung für die Probenkammern ausgestaltet und um eine vertikale Achse drehbar angeordnet. Es können Proben damit nacheinander von einer ersten Station, in welcher der Drehteller befüllt wird, an eine zweite Station mit einer Messvorrichtung für eine Analyse der Probe und an eine dritte Station für ein Entleeren und Reinigen übergeben werden. Mit einem weiteren Takt wird dann die gereinigte Probenkammer erneut an die erste Station für ein erneutes Befüllen übergeben. Es schließt sich ein weiterer Zyklus an, in dem alle benötigten Arbeitsschritte zur Analyse zeitgleich ausgeführt werden können.

In besonders bevorzugter Ausgestaltung kann die zumindest eine Probenkammer am äußeren Rand des Drehtellers integriert sein. Der Drehteller wird über eine zentrale Achse durch einen Antriebsmotor in Drehung versetzt. Am äußeren Rand angeordnete Probenkammern liegen möglichst weit von der Antriebseinheit in einem Bereich, in dem genügend Bauraum für weitere Einrichtungen zum Befüllen, Messen und Entleeren der Probenkammern vorhanden ist. Am äußeren Rand des Drehtellers können auch mehrere Probenkammern mit einem optimalen Aufnahmevolumen flächig ausgestaltet werden.

Vorteilhafterweise kann zwischen der ersten Station und der zweiten Station eine Abstreifeinrichtung zum Erzielen einer definierten Probenmenge angeordnet sein. Eine Abstreifeinrichtung entfernt überschüssiges Probenmaterial und kann gegebenenfalls die Probe auch verdichten. Zum Abstreifen überschüssigen Probenmaterials ist es ausreichend, den Drehteller unter einem unmittelbar an seiner Oberseite entlanggeführten Arm hindurchzudrehen. Der Arm kann dabei in radialer Richtung am Drehteller über eine Probenkammer hinwegreichen und außerhalb gehalten werden, so dass dieser über die Oberseite des Drehtellers streicht und mit seiner Vorderkante das überzählige Probenmaterial abzieht. Zum Verdichten kann die Abstreifeinrichtung klingenartige Gestalt aufweisen. Die Abziehklinge ist so angeordnet, dass diese Probenmaterial in die Ausnehmung der Probenkammer einstreicht und dabei verdichtet sowie überzähliges Probenmaterial entfernt, so dass insgesamt eine reproduzierbare, exakte Schichtdicke hergestellt wird.

In weiterer vorteilhafter Ausgestaltung der Erfindung kann die zweite Station aus mehreren Messeinrichtungen bestehen, welche in Drehrichtung hintereinander angeordnet sind. Zur chemischen Messung können auch mehrere Messmethoden zum Einsatz kommen, beispielsweise zur Bestimmung von Feuchte, Protein- und Fettgehalt von Produkten. Angedacht sind auch physikalische Messeinrichtungen, die den Einsatz von mehreren unterschiedlichen Messapparaturen erforderlich machen. Auch weitere optische Messeinrichtungen zur Kontrolle einer korrekt gefüllten Probenkammer können vorgeschaltet sein.

In weiterer ergänzender Ausführungsform kann in Drehrichtung vor der Messvorrichtung in der zweiten Station eine weitere Messvorrichtung zur Analyse der in einer Probenkammer befindlichen Probenmenge angeordnet sein. Im diesem Takt wird die so vorbereitete Probe mittels eines Detektors oder einer Kamera dahingehend untersucht, ob die gewünschte Probendicke eingestellt ist und eine zufriedenstellende Probenaufbereitung stattgefunden hat.

Vorteilhafterweise kann die zumindest eine Probenkammer vollständig innerhalb des Drehtellers ausgebildet sein. Die Probenkammern schließen dann mit der Ober- bzw. Unterseite des Drehtellers ab, so dass keine überstehenden Auskragungen vorhanden sind. Dies erleichtert das Hindurchdrehen des Drehtellers durch das Innere einer Integrationskugel. In im Drehteller vollständig integrierte flache Probenkammern können die Probensubstanzen als besonders dünne Schicht eingebracht werden. Hierbei ist angedacht, dass neben einer gleichförmigen Dicke der Schicht auch eine gleichmäßige Verdichtung der Substanz zu einer homogenen Verteilung führt. Zur Analyse werden, insbesondere zur Messung fester Substanzen, auf diese Weise in der jeweiligen Probenkammer geeignet dünne Messproben präpariert.

Zur Messung von Schichten unterschiedlicher Dicke kann insbesondere vorgesehen sein, Wechseleinsätze bereitzustellen, mit welchen sich unterschiedliche Tiefen von Probenkammern und damit unterschiedliche Schichtdicken realisieren lassen, auszubilden. Diese Wechseleinsätze können dann bei Bedarf in die Probenkammern in den Drehteller eingesetzt werden. In einer besonders einfachen Variante der Erfindung sind die Wechseleinsätze als einfache planparallele Platten unterschiedlicher Dicke ausgebildet, welche selbstverständlich transparent für die zur Messung verwendete Strahlung sind. Durch einfaches Einlegen dieser Platten kann die Tiefe der Probekammern und damit die Schichtdicke verringert werden. Typische Schichtdicken liegen im Bereich von bis zu 5 mm.

Bevorzugt können die Unterseite des Bodens und/oder die Oberseite eines Deckels der Probenkammer mit der Unterseite des Drehtellers bzw. der Oberseite des Drehtellers fluchten.

In vorteilhafter Ausgestaltung der Erfindung kann die zumindest eine Probenkammer in der ersten Station und/oder zweiten Station und/oder dritten Station gasdicht verschließbar sein. Bei einer chemischen Analyse gasförmiger oder leicht flüchtiger Stoffe können weitere Messvorrichtungen mit gasführenden Anschlüssen zur Probenkammer angeordnet sein. Der gasführende Anschluss muss dabei so mit der Probenkammer in Verbindung treten, dass beim Weiterdrehen des Drehtellers sich die Verbindung löst und wieder an die nachfolgende Probenkammer andockt. Hierzu können an sich übliche Dichtungsmaßnahmen vorgesehen sein. Bei brennbaren Gasen oder Dämpfen in gesundheitsschädlichen Konzentrationen sollten besondere Vorkehrungen bezüglich der Dichtheit getroffen werden.

Vorteilhafterweise kann die zumindest eine Probenkammer einen für optische Strahlung durchlässigen Bodenbereich und/oder Deckelbereich aufweisen. Besonders bevorzugt sind optisch transparente Silikatgläser. So kann eine Probenkammer optional mit einem Deckelelement nach dem Befüllen und vor dem Transport zur zweiten Station abgedeckt werden.

Vorteilhafterweise kann die Transportvorrichtung in einer Einhausung unter vorgebbaren Umgebungsbedingungen angeordnet sein. Innerhalb der Einhausung kann beispielsweise eine konstante Temperatur bzw. eine genau vorgegebene Luftfeuchtigkeit eingestellt werden. Darüber hinaus wird eine derartige Einhausung einen unkontrollierten Austritt von gegebenenfalls gesundheitsschädlichen Gasen an die Umgebung verhindern. Ebenso kann durch Inertgase einer Verunreinigung oder Veränderung der Messprobe wirksam vorgebeugt werden.

Nachfolgend wird die Erfindung anhand der Zeichnung exemplarisch erläutert. Es zeigt:

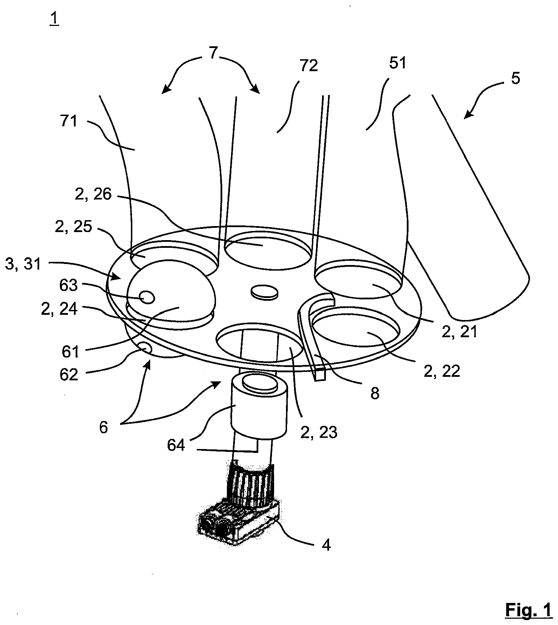

- Figur 1: Schematisch eine Ansicht einer erfindungsgemäßen Vorrichtung für die automatisierte Analyse von Feststoffen oder Fluiden.

Fig. 1 zeigt schematisch eine Ansicht einer Vorrichtung 1 für die automatisierte Analyse von Feststoffen oder Fluiden. Diese beinhaltet eine erste Station 5 mit einer Dosiereinheit 51 für das nacheinander getaktete Befüllen von, in diesem Fall, sechs Probenkammern 2 mit einer vorgegebenen Probenmenge. Eine zweite Station 6 beinhaltet ein Kugelmesssystem 61 für eine Analyse der in einer Probenkammer 2 befindlichen Probe sowie eine Kamera als weitere vorgeschaltete Messvorrichtung 64. Eine dritte Station 7 ist eine Entleervorrichtung 71 und eine Reinigungsvorrichtung 72 für die jeweiligen Probenkammern 2. Zudem ist eine Transportvorrichtung 3 als Drehteller 31 für einen umlaufenden Transport der Probenkammern 2 von einer Station zur nächsten ausgeführt. Nach einer vollständigen Drehung wird die erste Station 5 erneut zum Füllen der gereinigten Probenkammern 2 erreicht. Erfindungsgemäß ist die Messvorrichtung 61 der zweiten Station 6 eine Integrationskugel, durch deren Inneres jede der Probenkammern 2 hindurchführbar ist.

In Fig. 1 ist zwischen der ersten Station 5 und der zweiten Station 6 eine Abstreifeinrichtung 8 montiert. Diese Abstreifeinrichtung 8 umfasst einen auf der Oberseite des Drehtellers 31 entlang geführten Arm, der überzähliges Probenmaterial, welches über die flache Probenkammer 2 hinaussteht abzieht. Die weiteren Halterungen des Arms außerhalb des Drehtellers 31 sind der Einfachheit halber nicht dargestellt.

Die sechs Probenkammern 2 sind im Drehteller 31 am Umfang im Winkelabstand von jeweils 60° angeordnet. Mittels eines Antriebsmotors 4 kann der Drehteller 31 schrittweise um eine vertikale Achse weitergedreht werden. Im Drehteller ist in dieser bevorzugten Ausführungsform eine erste Probenkammer 21 bei der ersten Station 5 zum Befüllen positioniert. Hier werden beispielsweise Milchpulver, Getreide oder sonst schütt- oder fließfähige Güter, eingebracht. Optional kann in dieser Position die Messprobe durch einen, in der Fig. 1 nicht dargestellten, von oben geführten Stempel verdichtet werden. Die zweite Probenkammer 22 befindet sich in Drehrichtung unmittelbar vor dem Arm der Abstreifeinrichtung 8. Die dritte Probenkammer 23 befindet sich in der zweiten Station 6 und wird mittels einer Kamera als weitere Messvorrichtung 64 auf eine ausreichend gute Probenqualität hin untersucht.

Ebenfalls an der zweiten Station 6 befindet sich die vierte Probenkammer 24 innerhalb der Integrationskugel als Messvorrichtung 61 für eine chemische Analyse der Probe. In der Integrationskugel erfolgt dann beispielsweise eine Transmissionsmessung. Die Integrationskugel 61 besteht dabei aus einer aus zwei Halbschalen aufgebauten Photometerkugel, in deren Äquatorialebene die jeweilige Probenkammer taktweise eingebracht wird. An der unteren Halbschale der Integrationskugel 61 befindet sich ein Eintrittsfenster 62 für die Messstrahlung. Die obere Halbschale der Integrationskugel 61 weist ein Austrittsfenster 63 auf, durch welches die Messstrahlung zu einer Detektoreinheit gelangen kann. Detektoreinheit und die außerhalb der Integrationskugel 61 befindlichen weiteren optischen Abbildungseinheiten und Filter sind in Fig. 1 der Einfachheit halber nicht dargestellt. Diese zusätzlichen Einrichtungen sind prinzipiell bekannt und beispielsweise aus dem vorstehend zitierten Stand der Technik für den Fachmann zu entnehmen.

Die fünfte Probenkammer 25 ist unter einem Absaugrohr der Entleervorrichtung 71 der dritten Station 7, über welches das Probenmaterial weitgehend bis auf geringfügige Reste entfernt wird. Die sechste Probenkammer 26 befindet sich in der dritten Station 7 am Ort der Reinigungsvorrichtung 72, in der die sechste Probenkammer 26 vollständig gereinigt wird, damit diese in die erste Station 5 zum Befüllen weitergedreht werden kann. Insgesamt weist folglich der Drehteller genau so viele Probenkammern auf, wie insgesamt Einrichtungen in den Stationen im Messzyklus zur Präparation und Messung für einen Durchlauf vorhanden sind.

### Bezugszeichenliste:

- 1: Vorrichtung

- 2: Probenkammer
- 21: erste Probenkammer
- 22: zweite Probenkammer
- 23: dritte Probenkammer
- 24: vierte Probenkammer
- 25: fünfte Probenkammer
- 26: sechste Probenkammer

- 3: Transportvorrichtung
- 31: Drehteller

- 4: Antriebsmotor

- 5: erste Station
- 51: Dosiereinheit

- 6: zweite Station
- 61: Messvorrichtung, Integrationskugel
- 62: Eintrittsfenster
- 63: Austrittsfenster
- 64: weitere Messvorrichtung, Kamera

- 7: dritte Station
- 71: Entleervorrichtung
- 72: Reinigungsvorrichtung

- 8: Abstreifeinrichtung

## Patentansprüche

1. Vorrichtung (1) für die automatisierte Analyse von Feststoffen oder Fluiden, umfassend:
- eine erste Station (5) mit einer Dosiereinheit (51) für das Befüllen zumindest einer Probenkammer (2) mit einer vorgegebenen Probenmenge,
- eine zweite Station (6) mit zumindest einer Messvorrichtung (61) für eine Analyse der in einer Probenkammer (2) befindlichen Probe und
- eine dritte Station (7) mit einer Entleer- und Reinigungsvorrichtung (71, 72) für die zumindest eine Probenkammer (2), sowie
- eine Transportvorrichtung (3) für einen umlaufenden Transport der zumindest einen Probenkammer (2) von einer Station zur nächsten, bis die erste Station (5) erneut erreicht ist,
**dadurch gekennzeichnet, dass**
die Messvorrichtung (61) der zweiten Station (6) ein Kugelmesssystem ist, welches eine aus zwei Halbschalen ausgebildete Photometerkugel umfasst, in deren Äquatorialebene die zumindest eine Probenkammer (2) taktweise einbringbar und hindurchführbar ist.

2. Vorrichtung (1) nach Anspruch 1, **dadurch gekennzeichnet, dass**
die Transportvorrichtung (3) einen Drehteller (31) umfasst, in dem eine Probenkammer (2) integriert ist oder mehrere Probenkammern (21, 22, 23, 24, 25, 26) integriert sind.

3. Vorrichtung (1) nach Anspruch 2, **dadurch gekennzeichnet, dass**
die zumindest eine Probenkammer (2) am äußeren Rand des Drehtellers (31) integriert ist.

4. Vorrichtung (1) nach einem der Ansprüche 1 bis 3, **dadurch gekennzeichnet, dass**
zwischen der ersten Station (5) und der zweiten Station (6) eine Abstreifeinrichtung (8) zum Erzielen einer definierten Probenmenge angeordnet ist.

5. Vorrichtung (1) nach einem der Ansprüche 2 bis 4,
**dadurch gekennzeichnet, dass**
die zweite Station (6) aus mehreren Messeinrichtungen besteht, welche in Drehrichtung hintereinander angeordnet sind.

6. Vorrichtung (1) nach einem der Ansprüche 2 bis 5,
**dadurch gekennzeichnet, dass**
in Drehrichtung vor der Messvorrichtung (61) in der zweiten Station (6) eine weitere Messvorrichtung (64) zur Analyse der in einer Probenkammer (2) befindlichen Probenmenge angeordnet ist.

7. Vorrichtung (1) nach einem der Ansprüche 2 bis 6,
**dadurch gekennzeichnet, dass**
die zumindest eine Probenkammer (2) vollständig innerhalb des Drehtellers (31) ausgebildet ist.

8. Vorrichtung (1) nach einem der Ansprüche 2 bis 7,
**dadurch gekennzeichnet, dass**
Wechseleinsätze vorhanden sind, mit welchen sich unterschiedliche Tiefen von Probenkammern (2) realisieren lassen.

9. Vorrichtung (1) nach einem der Ansprüche 2 bis 8,
**dadurch gekennzeichnet, dass**
die Unterseite des Bodens und/oder die Oberseite des Deckels der Probenkammer (2) mit der Unterseite des Drehtellers (31) bzw. der Oberseite des Drehtellers (31) fluchtet.

10. Vorrichtung (1) nach einem der Ansprüche 1 bis 9,
**dadurch gekennzeichnet, dass**
die zumindest eine Probenkammer (2) in der ersten Station (5) und/oder zweiten Station (6) und/oder dritten Station (7) gasdicht verschließbar ist.

11. Vorrichtung (1) nach einem der Ansprüche 1 bis 10,
**dadurch gekennzeichnet, dass**
die zumindest eine Probenkammer (2) einen für optische Strahlung durchlässigen Bodenbereich und/oder Deckelbereich aufweist.

12. Vorrichtung (1) nach einem der Ansprüche 1 bis 11,
**dadurch gekennzeichnet, dass**
die Transportvorrichtung (3) in einer Einhausung unter vorgebbaren Umgebungsbedingungen angeordnet ist.

## Claims

1. Device (1) for the automated analysis of solids or fluids, comprising:
- a first station (5) having a metering unit (51) for the filling of at least one sample chamber (2) with a specified sample quantity,
- a second station (6) having at least one measurement device (61) for an analysis of the sample situated in a sample chamber (2) and
- a third station (7) having an emptying device and cleaning device (71, 72) for the at least one sample chamber (2), and also
- a transport device (3) for a revolving transport of the at least one sample chamber (2) from one station to the next until the first station (5) is reached again,
**characterized in that**
the measurement device (61) of the second station (6) is a spherical measurement system comprising a photometer sphere formed from two half-shells, into/in the equatorial plane of which it is possible to introduce and guide the at least one sample chamber (2) phasically.

2. Device (1) according to Claim 1, **characterized in that**
the transport device (3) comprises a rotary plate (31) in which one sample chamber (2) is integrated or multiple sample chambers (21, 22, 23, 24, 25, 26) are integrated.

3. Device (1) according to Claim 2, **characterized in that**
the at least one sample chamber (2) is integrated on the outer edge of the rotary plate (31).

4. Device (1) according to any of Claims 1 to 3,
**characterized in that**
a wiper mechanism (8) for achieving a defined sample quantity is arranged between the first station (5) and the second station (6).

5. Device (1) according to any of Claims 2 to 4,
**characterized in that**
the second station (6) consists of multiple measurement mechanisms which are arranged one after the other in the direction of rotation.

6. Device (1) according to any of Claims 2 to 5,
**characterized in that**
a further measurement device (64) for the analysis of the sample quantity situated in a sample chamber (2) is arranged, in the direction of rotation, before the measurement device (61) in the second station (6).

7. Device (1) according to any of Claims 2 to 6,
**characterized in that**
the at least one sample chamber (2) is completely formed within the rotary plate (31).

8. Device (1) according to any of Claims 2 to 7,
**characterized in that**
there are interchangeable inserts, by means of which it is possible to realize different depths of sample chambers (2).

9. Device (1) according to any of Claims 2 to 8,
**characterized in that**
the lower side of the base and/or the upper side of the lid of the sample chamber (2) aligns with the lower side of the rotary plate (31) and the upper side of the rotary plate (31), respectively.

10. Device (1) according to any of Claims 1 to 9,
**characterized in that**
the at least one sample chamber (2) is closable in a gas-tight manner in the first station (5) and/or second station (6) and/or third station (7).

11. Device (1) according to any of Claims 1 to 10,
**characterized in that**
the at least one sample chamber (2) has a base region and/or lid region that is penetrable for optical radiation.

12. Device (1) according to any of Claims 1 to 11,
**characterized in that**
the transport device (3) is arranged in a housing under specifiable environmental conditions.

## Revendications

1. Dispositif (1) d'analyse automatisée de matières solides ou de fluides, comportant :
- une première station (5) dotée d'une unité de dosage (51) pour le remplissage d'au moins une chambre d'échantillon (2) avec une quantité prédéfinie d'échantillon,
- une deuxième station (6) dotée d'au moins un dispositif de mesure (61) pour une analyse de l'échantillon se trouvant dans une chambre d'échantillon (2) et
- une troisième station (7) dotée d'un dispositif de vidage et de nettoyage (71, 72) pour l'au moins une chambre d'échantillon (2), ainsi que
- un dispositif de transport (3) pour un transport rotatif de l'au moins une chambre d'échantillon (2) d'une station à la suivante, jusqu'à ce que la première station (5) soit à nouveau atteinte,
**caractérisé en ce que**
le dispositif de mesure (61) de la deuxième station (6) est un dispositif de mesure sphérique, lequel comporte une sphère photométrique formée à partir de deux demi-coques dans les plans équatoriaux desquelles l'au moins une chambre d'échantillon (2) peut être introduite et guidée de manière cadencée.

2. Dispositif (1) selon la revendication 1, **caractérisé en ce que**
le dispositif de transport (3) comporte une plaque rotative (31) dans laquelle une chambre d'échantillon (2) est intégrée ou plusieurs chambres d'échantillon (21, 22, 23, 24, 25, 26) sont intégrées.

3. Dispositif (1) selon la revendication 2, **caractérisé en ce que**
l'au moins une chambre d'échantillon (2) est intégrée sur le bord extérieur de la plaque rotative (31).

4. Dispositif (1) selon l'une des revendications 1 à 3,
**caractérisé en ce**
**qu'**un dispositif de raclage (8) servant à obtenir une quantité définie d'échantillon est disposé entre la première station (5) et la deuxième station (6).

5. Dispositif (1) selon l'une des revendications 2 à 4,
**caractérisé en ce que**
la deuxième station (6) est constituée de plusieurs dispositifs de mesure, lesquels sont disposés les uns derrière les autres dans le sens de rotation.

6. Dispositif (1) selon l'une des revendications 2 à 5,
**caractérisé en ce**
**qu'**un autre dispositif de mesure (64) servant à l'analyse de la quantité d'échantillon se trouvant dans une chambre d'échantillon (2) est disposé avant le dispositif de mesure (61) dans la deuxième station (6) dans le sens de rotation.

7. Dispositif (1) selon l'une des revendications 2 à 6,
**caractérisé en ce que**
l'au moins une chambre d'échantillon (2) est formée complètement à l'intérieur de la plaque rotative (31).

8. Dispositif (1) selon l'une des revendications 2 à 7,
**caractérisé en ce que**
des inserts interchangeables sont prévus, à l'aide desquels différentes profondeurs de chambres d'échantillon (2) peuvent être réalisées.

9. Dispositif (1) selon l'une des revendications 2 à 8,
**caractérisé en ce que**
le côté inférieur du fond et/ou le côté supérieur du couvercle de la chambre d'échantillon (2) sont en affleurement avec le côté inférieur de la plaque rotative (31) ou le côté supérieur de la plaque rotative (31).

10. Dispositif (1) selon l'une des revendications 1 à 9,
**caractérisé en ce que**
l'au moins une chambre d'échantillon (2) peut être fermée de manière étanche aux gaz dans la première station (5) et/ou la deuxième station (6) et/ou la troisième station (7) .

11. Dispositif (1) selon l'une des revendications 1 à 10,
**caractérisé en ce que**
l'au moins une chambre d'échantillon (2) comprend une région de fond et/ou une région de couvercle transparente(s) au rayonnement optique.

12. Dispositif (1) selon l'une des revendications 1 à 11,
**caractérisé en ce que**
le dispositif de transport (3) est disposé dans une enceinte dans des conditions ambiantes pouvant être prédéfinies.
